# EUROPEAN PATENT APPLICATION

(11) **EP 4 125 095 A1**
(43) Date of publication of application: **01.02.2023**
(21) Application number: 22187065.2
(22) Date of filing: 26.07.2022
(51) Int. Cl.: G16H 15/00

(54) **SYSTEM AND METHOD FOR DATA PROCESS**

(30) Priority: 27.07.2021 TW 110127614
(71) Applicant: HowiseAI International Co., Ltd., 24449 New Taipei City (TW)
(72) Inventor: Chang, Ru-Yng, 24449 New Taipei City (TW)
(74) Representative: Betten & Resch

(57) **Abstract**

A system for data process comprises an operating platform for storing and reading a data unit. A data processing module signally connected to the operating platform. The data unit is structured or unstructured. The data processing module labeling and processing the data unit, and generating a visualization diagram. The system for data process includes a graphical user interface, which can achieve one of the purposes of this present disclosure of improving the data visualization of structured data and unstructured data.

## Description

### CROSS REFERENCE TO RELATED APPLICATION

The present application claims priority to Taiwan Patent Application Serial No. 110127614, filed on July 27,2021, which is hereby incorporated by reference in their entirety.

### FIELD

The present disclosure relates to a system and a method for data process, more particularly to a system and a method for data process used for visualizing data.

### BACKGROUND

An unstructured data and a semi-structured data can be written freely, which can ensure that the data is more abundant and complete. However, for computers, unstructured data and semi-structured data are more difficult to be recognized than structured data. Therefore, the existing natural language processing (NLP) is to improve the computer's understanding of unstructured and semi-structured data, and further to be able to apply to the unstructured and semi-structured data.

NLP has been developed and applied quite maturely. It includes the application that can automatically extract unstructured text or semi-structured text and recognize its semantic category. This means that one of the NLP applications can transform the content of the text into semantically structured data. In this way, personnel may use the structured data for further processing, statistics, analysis and application.

NLP is more conducive to data analysis and understanding in various professional fields, and can also assist industries in making decision and judgments, deriving various benefits, such as: retail, finance, medical care... etc. Specifically, the application of NLP in the medical field can assist in understanding various data trends and statistical analysis of patients, diseases, etc. from various clinical records and medical literature. In addition, various analyses are more helpful to medical research, which in turn affects clinical decision-making, reduces misdiagnosis and avoids unnecessary medical treatments.

However, in the processing and application stage, because NLP needs to comprehend interdisciplinary knowledge including the knowledge of linguistics and information science with a higher threshold to learn, it is difficult for non-IT (information technology) personnel to get started. In addition, the natural language analysis tools for non-IT personnel are very rare, which hinders the progress of natural language applications to industries. Taking the medical field as an example, the application of NLP in this field is urgently to be improved due to the diverse data content and data types in the medical field. For example, the content of a "medical record of out-patient service" may be symptoms, symptom duration, symptom severity, personal disease history, etc., which usually are stored in unstructured data formats. Similarly, a "patient examination report" stored in unstructured format often records the findings/diseases, the size of the findings/diseases, the time, and so on. Conversely, the gender, date of birth, educational background, etc., often recorded in the patient's background information, are usually stored in a structured data format. Accordingly, the problem is that it is difficult for non-IT personnel to utilize the aforementioned diverse data format content in lack of available NLP tools.

In addition, NLP can assist personnel in professional fields to achieve the purpose of data analysis. However, to achieve the goal, operators who perform NLP and analysis need to be capable of making decisions with professional knowledge in the field. Also, different roles may need to perform the analysis in different aspects. For example, the data to be analyzed and observed to a physician or a pharmacist in the medical care field may be different; the data which a doctor wishes to observe or analyze is also different among different doctors. The point is that, given the gap in the background and demands of operators, the field of NLP still needs inventions that can solve the aforementioned problems.

### SUMMARY OF THE DISCLOSURE

A system for data process, comprising: an operating platform for storing and reading a data unit; a data processing module signally connected to the operating platform; the data unit is structured or unstructured; the data processing module labelling and processing the data unit, and generating a visualization diagram.

A method for data process, a data processing module is used to process one of unstructured data and structured data, to process at least one data unit, and perform the following steps: (A) a step for project creation: an operating platform generates a project data set for accessing the at least one data unit including a plurality of data values; the project data set includes at least one data attribute; (B) a step for semantic labeling decision and labeling process: the data processing module processes the at least one data unit, determines a semantic labeling decision, and outputs a labelled-up data unit, the semantic labeling decision makes one of the plurality of data values to correspond to the at least one data attribute; when the at least one data unit is unstructured data format, the data processing module performs a step for automatic semantic labeling to complete the semantic labeling decision makes one of the plurality of data values to correspond to the at least one data attribute; (C) a step for data unit storage: the data processing module stores the labelled-up data unit to the project data set; (D) a step for process and output: the data processing module processes the project data set to generate a visualization diagram.

### BRIEF DESCRIPTION OF THE DRAWINGS

FIG. 1 is a block diagram I of the first embodiment of the present disclosure.
FIG. 2 is a block diagram II of the first embodiment of the present disclosure.
FIG. 3 is a block diagram I of the second embodiment of the present disclosure.
FIG. 4 is a block diagram II of the second embodiment of the present disclosure.
FIG. 5 is a block diagram of the third embodiment of the present disclosure.
FIG. 6 is a block diagram of a visualization diagram editing interface of the third embodiment of the present disclosure.
FIG. 7 is an illustration of a data unit in the third embodiment.
FIG. 8 is a block diagram of the fourth embodiment of the present disclosure.
FIG. 9a is a variation of the fourth embodiment of the present disclosure.
FIG. 9b is an example diagram showing the result of co-occurrence analysis in the fourth embodiment of the present disclosure.
FIG. 9c is an illustration of a variation of the fourth embodiment of the present disclosure.
FIG. 10 is a block diagram of the fifth embodiment of the present disclosure.
FIG. 11 is a block diagram I of the sixth embodiment of the present disclosure.
FIG. 12 is a flowchart of a method for data labeling according to the sixth embodiment of the present disclosure.
FIG. 13a is a flowchart of a data processing method disclosed in this disclosure.
FIG. 13b is a block diagram of a system for data processing of the present disclosure.
FIG. 13c is a flowchart of another method for data process of the present disclosure. FIG. 13d is a flowchart of another method for data process of the present disclosure.

### DETAILED DESCRIPTION

According to an implementation there is provided a system for data process comprising an operating platform for storing and reading a data unit. A data processing module can be signally connected to the operating platform. The data unit can be structured or unstructured. The data processing module can be adapted to labeling and processing the data unit, and generating a visualization diagram. The system for data process can include a graphical user interface, which can achieve one of the purposes of this present disclosure of improving the data visualization of structured data and unstructured data. The term "signally connected" used in the detailed description of the present disclosure (including the scope of the claims) can refer to any direct or indirect connection means. For example, if the text describes that a data processing module signally connected to an operating platform, it should be interpreted as that the data processing module can be directly connected to the operating platform, or the data processing module can be connected by other devices or certain connection means, indirectly connected to the operating platform. In addition, wherever possible, elements/components/steps with the same reference numerals in the drawings and embodiments represent the same or similar parts. Elements/components/steps that use the same reference numerals or use the same terms in different embodiments may refer to related descriptions.

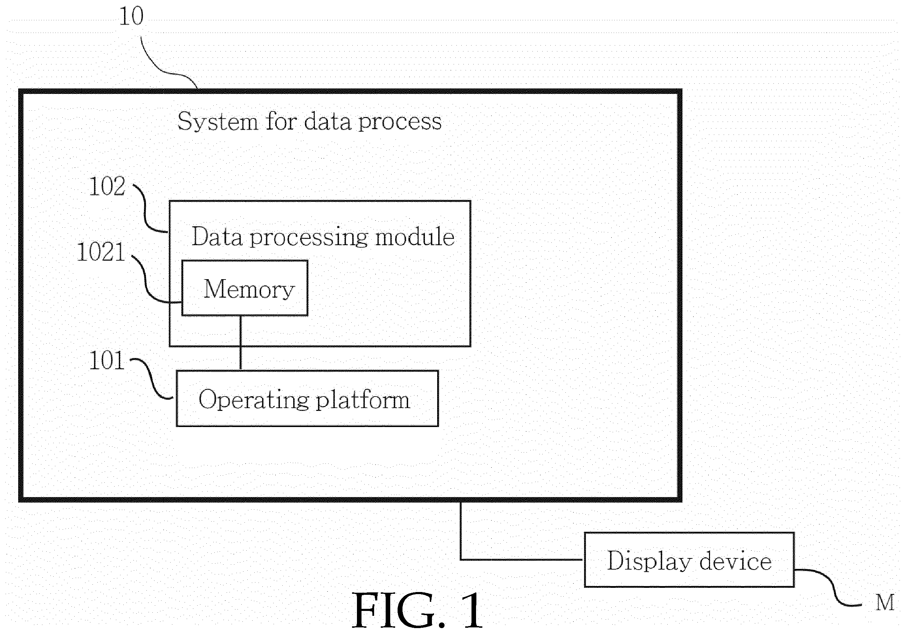

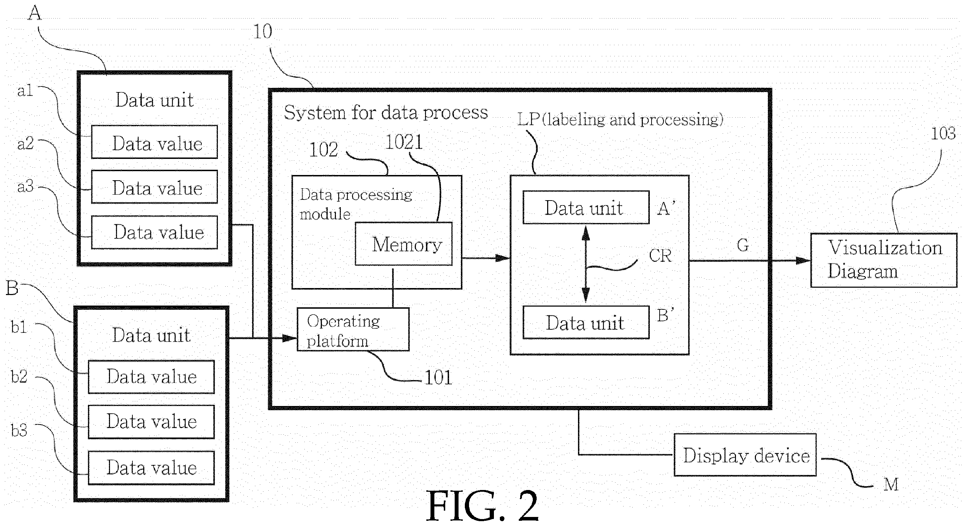

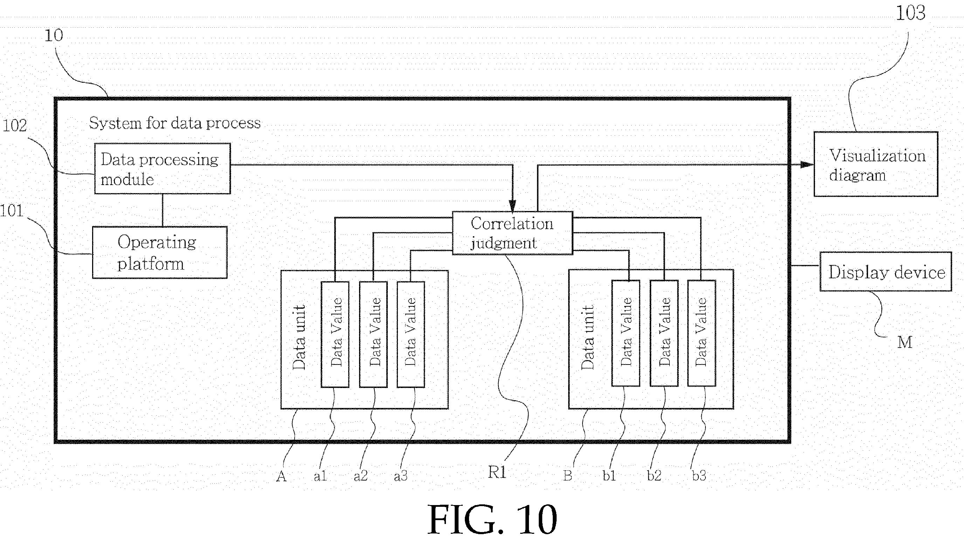

Refer to FIG. 1 to FIG. 2, which are block diagrams of the first embodiment of the present disclosure. The system for data process **10** of this embodiment can process data unit **A,** data unit **B,** or both, and generate a visualization diagram **103.** The system for data process **10** mainly includes an operating platform **101** signally connected to a data processing module **102,** the data processing module **102** includes a memory **1021,** and the data processing system **10** is signally connected to a display device **M,** wherein:
(1) Data unit **A** and data unit **B** can be in a structured data format or an unstructured data format. If data unit **A** and data unit **B** are stored in a structured data format, it means that data unit **A** and data unit **B** already contain data attributes, fixed fields, specific order, or other scheme used to classify data records or data values. For example, file formats commonly used to record structured data formats such as csv, xls and other file formats. Taking the medical industry as an example, examples that often recorded in structured data formats are: patient data, medication record, drug data, medicine data (pharmaceutical raw materials), doctor's advice data, doctor's data, equipment data, department data, hospitalization data, specialty data, examination data, shift report, etc.
(2) If data unit A and data unit B are stored in unstructured data format, it means that the data unit is not stored in the form of data attributes, fixed fields, or specific order. Examples of unstructured data formats are: original is text data, including news reports, medical records, community forum posts, texts of reports, emails, etc. or other similar texts; or speech-to-text texts, including from sources of: telephone customer service, meeting recordings, or other similar texts; or even PDF files, pictures and video data with texts, including: subtitles, graphic works, or other similar data. Among them, unstructured data also refers to semi-structured data, that is, a data unit contains a part of data stored in structured data format. For example, the file formats commonly used to record semi-structured data formats are such as JSON, XML, HTML and other file formats. File formats commonly used to record unstructured data formats such as PNG, PDF and other file formats. However, the structured data format or unstructured data format referred to in the present disclosure is not limited to the previous disclosed examples. Taking the medical industry as an example, examples often recorded in unstructured data formats are: unstructured text, medical record, doctor's advice data, patient data, medication record, medicine (pharmaceutical raw materials) profile data, drug profile data, doctor's data, equipment data, and department data, hospitalization data, examination data, shift report, etc.
(3) The operating platform **101** is for a system user of the system for data process **10,** and the operating platform can be presented on a display device M with a graphical user interface (GUI). The operating platform 101 is for the system user to store the data unit **A,** the data unit **B,** or both in the memory **1021;** or to read the data unit **A,** the data unit **B,** or both from the memory **1021.** The memory **1021** can be a scratch pad memory or a storage device such as a hard disk.
(4) The operating platform **101** is for the system user to import data unit **A,** data unit **B,** or both and store them in the memory **1021.** The operating platform **101** is for the system user to give an execution command to the data processing module **102.** The operating platform **101** includes a GUI for the system user to create a relationship between the data unit **A** and the data unit **B.** Specifically, it can be implemented by applying the primary key and foreign key in the concept of a relational database. The operating platform **101** provides a graphical user interface, allowing the system user to establish a key between any two of a plurality of data units by clicking.
(5) The data processing module **102** is used to "label" and "process" the data unit **A,** the data unit **B,** or both which are stored in the memory **1021,** and generates a visualization diagram **103.** The system for data process **10** is used for transmitting the visualization diagram **103** to the display device **M** for viewing by the system user.
(6) When the data processing module **102** executes "labeling", the data processing module **102** labels the text content of the data unit **A,** the data unit **B,** or both. The labelled data unit **A** and data unit **B** are labelled as labelled data unit **A'** and labelled data unit **B'** in the FIG. 2. The labelled text, value, or data in the labelled data unit **A'** and the labelled data unit **B'** is stored in a structured data format and used as a material for generating the visualization diagram **103.**
(7) This embodiment includes two "labeling" means: one is that the system for data process **10** can be used by the system user to perform manual labeling with the operating platform **101.** To further illustrate, the manual labeling includes: the system user gives a labeling execution command by the operating platform **101** to make the data processing module **102** label the text content of the data units **A** and **B.** Another "labeling" mean is that the data processing module **102** can automatically label the data units **A** and **B.** To further illustrate, in order to achieve the function of automatic labeling, the data processing module 102 includes an automatic data labeling module (as an embodiment disclosed in FIG. 10) to automatically identify the text content of the data units **A** and **B,** and execute the labeling.
(8) After the data processing module **102** executes "labeling", it "processes" the labelled data unit **A',** the labelled data unit **B',** or both, and generates a visualization diagram **103.** The data processing module **102** may include, for example, Matplotlib, Pyecharts, Plotly, Bokeh, Seaborn, Python-based visualization plug-ins, R language-based visualization plug-ins, or other database-based visualization tools to process labelled data units **A',** labelled data unit **B',** or both. To further illustrate, when the data processing module **102** performs "processing", a data correlation CR of the labelled data unit **A'** and the labelled data unit **B'** is created, and a visualization diagram **103** is generated based on the data correlation **CR;** wherein, the data correlation **CR** may be a relationship key or a foreign key created between the data unit **A** and the data unit **B,** which the key and the foreign key is used to create data correlation between the data unit **A** and the data unit **B.**

As aforesaid, when the data units **A** and **B** stored (or temporarily stored) in the memory **1021** of the operating platform **101** are unstructured data units, the operating platform **101** allows the system user to choose whether to perform manual labeling the data units **A** and **B** by provided GUI on the operating platform **101** or automatic labeling the data **A** and **B** by the data processing module **102.** When the system user performs manual labeling, the operating platform 101 allows the system user to determine or edit a label of the data unit **A** and the data unit **B.**

This embodiment can be applied to both the data unit **A** and the data unit **B** are in a structured data format or an unstructured data format; or it can be applied to the two data units, where one of the data unit **A** and the data unit **B** is in the unstructured date format. As a result, one of the effects achieved by this embodiment: system users can easily operate the data unit **A** and the data unit **B,** which in different data formats, by the operating platform 101 to achieve the purpose of visualizing data, wherein this embodiment provides a better user experience; also, it is easy for users to use and good for introducing to various industry categories.

In another embodiment, the data unit **A,** the data unit **B,** or both may be a labelled data unit **A'** or a labelled data unit **B'** after performing "labeling" in advance. In this way, the previously labelled text content can be imported to the data processing module **102** by the operating platform **101** and enable the data processing module **102** to process and generate the visualization diagram **103.** Thereby, the data processing module **102** can save labeling time.

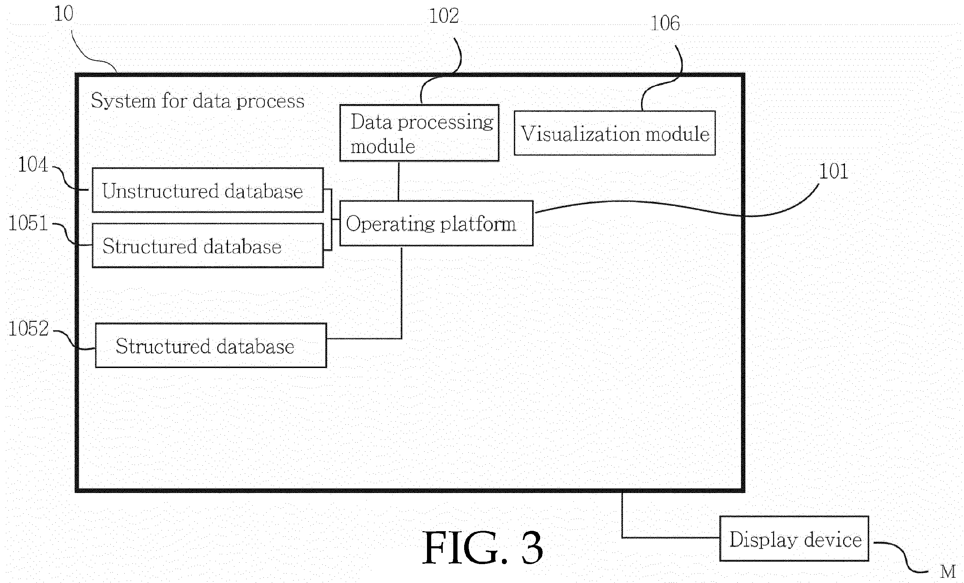

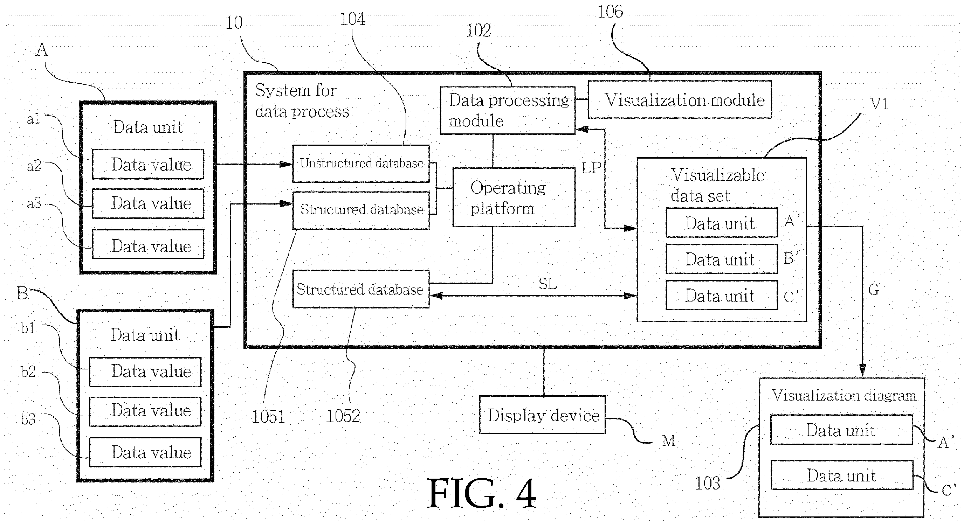

Please refer to FIGS. 3 and 4 for block diagrams of the second embodiment of the present disclosure. The system for data process **10** includes a visualization module **106,** an unstructured database **104,** a structured database **1051,** and a structured database **1052** signally connected the operation platform **101.**

The visualization module **106** signally connected the data processing module **102,** may include, for example, Matplotlib, Pyecharts, Plotly, Bokeh, Seaborn, Python-based visualization plug-ins, R language-based visualization plug-ins, or other database-based visualizations tools.

The unstructured database **104** is used to store the data unit **A** in an unstructured data format. The structured database **1051** is used to store the data unit **B** in the structured data format. Specifically, the database can be a database management system similar to MySQL, MSSQL, Apache Hbase, etc.

The operating platform **101** can provide a system user to access the data unit **A** from the unstructured database **104** or the data unit **B** from the structured database **105.**

The data processing module **102** is used to process the data unit **A** and the data unit **B,** and can output a visualizable data set **V1.** The system user can even import the visualizable data set **V1** to the data processing module **102** by the operating platform **101.** The visualization module **102** generates a visualization diagram **103** according to the visualization data set **V1.**

The visualizable data set **V1** includes one or more than one data unit that is able to be visualized by the data processing module **102,** for example, data units in a structured data format. In this embodiment, the visualizable data set **V1** includes a labelled data unit **C',** which is labelled by the data processing module **102** in advance. The data processing module **102** can copy or move the labelled data unit **A'** or the labelled data unit **B'** to the visualizable data set **V1.**

The system user can use the operating platform **101** to enable the data processing module **102** to process the labelled data unit **A',** the labelled data unit **B',** the labelled data unit **C',** or a combination thereof in the visualizable data set **V1.** Thereby, when the system user operates the operating platform **101,** the data processing module **102** can read one or more than one of the plurality of data units in the visualizable data set **V1,** and generate a visualization diagram **103.** To achieve aforesaid purpose, the visualizable data set **V1** can be stored in the structured database **1052.** The data processing module **102** processes the labelled data unit **A'** and the labelled data unit **C'** in the visualizable data set **V1** to generate the visualization diagram **103.**

The structured database **1052** of this second embodiment can be used to store and read the visualization data set **V1.** In this way, the data units that have been labelled in advance or the data units that are labelled and processed **LP** can be stored in the structured database **1052** by the visualizable data set **V1.** The operating platform **101** can read and import the visualizable data set **V1** to the data processing module **102** by the structured database **1052,** in order to output the visualization diagram **103.** In this way, the operations of a system user can be simplified, and the processing time required by the system can be shortened. In another variation of the second embodiment, the data unit that has been labelled or the data unit that has been labelled and processed **LP** can be stored as a computer-readable file, such as JSON; it is not a requirement to be stored in a structured database or unstructured the database.

In another variation of the second embodiment, the structured database **1051** and the structured database **1052** may be the same one.

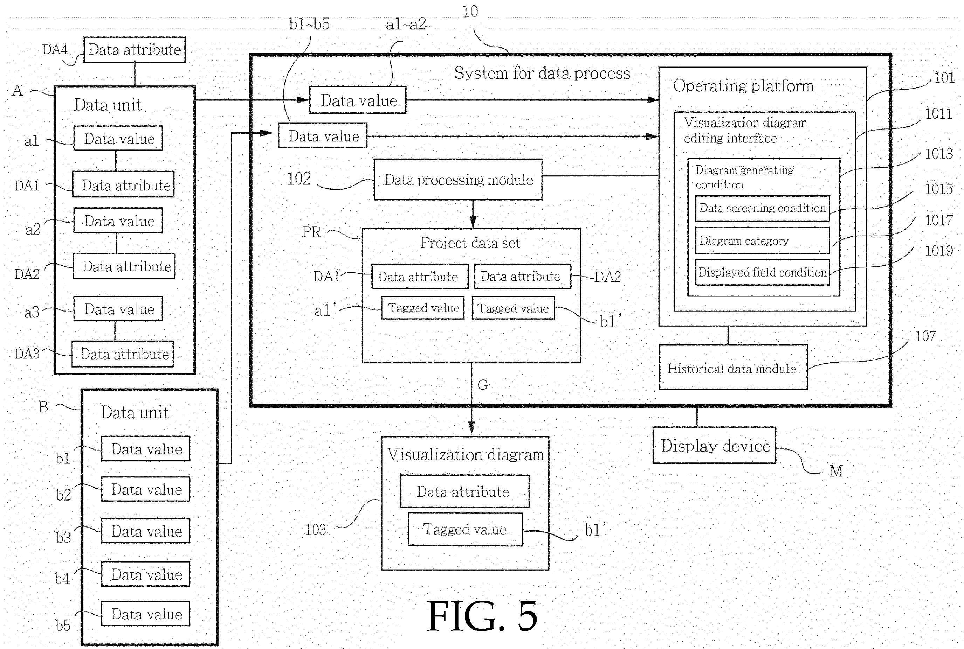

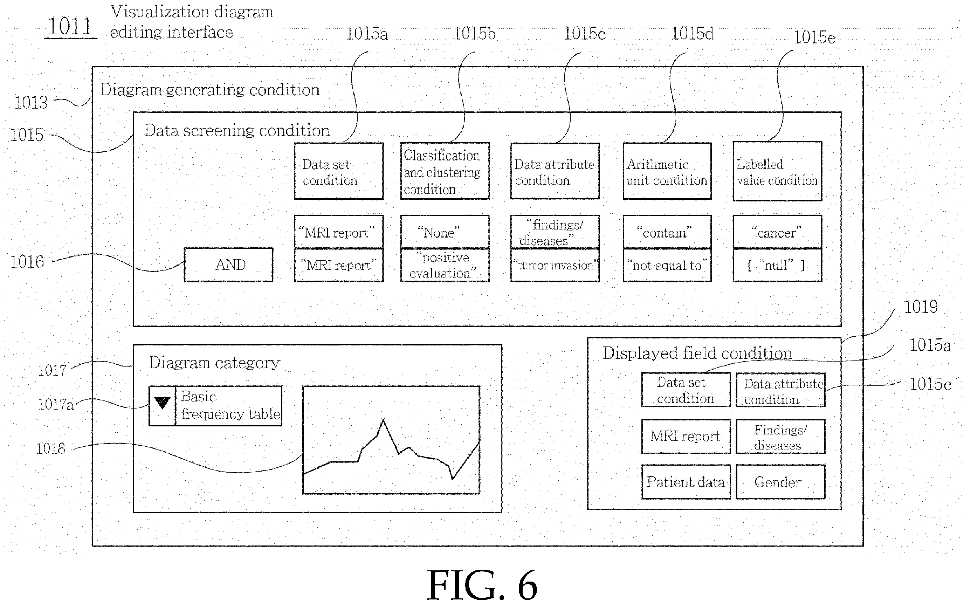

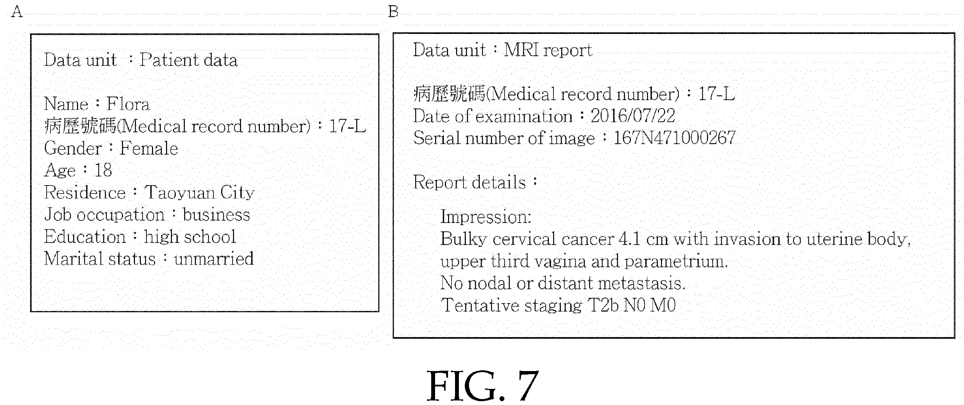

Next, please refer to FIG. 5 for a block diagram of the third embodiment of the present disclosure. FIG. 6 is a block diagram of a visualization diagram editing interface of the third embodiment of the present disclosure. FIG. 7 is an illustration of a data unit in the third embodiment. The operating platform **101** may include a visualization diagram editing interface **1011,** which is used for setting a generation condition of the visualization diagram **103.** The system for data process **10** includes a historical data module **107** signally connected to an operating platform **101** and a data processing module **102.** The system user can operate the operating platform **101** to generates a project data set **PR.** The operating platform **101** can edit and revise the project data set **PR.** The project data set **PR** can be temporarily stored or stored in the system for data process 10.

### Data value

In the third embodiment, the data unit **A** is a structured data format, such as patient data. The data unit **B** is an unstructured data format, such as an MRI report. The data unit **A** includes data values **a1** to **a3**, and the data unit **B** includes data values **b1** to **b5.**

The data values **a1** to **a3** can be stored in the data unit **A** in a structured data format. Specifically, the data value refers to a value stored in a storage field in a data unit, such as a character, a word, a value, a bit value, etc. For example, if the data unit is patient data in a structured data format, the data value can be a record or a tuple in the data unit, such as "name," "Flora(name)," " 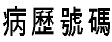 (Medical record number)," "17-L." In this case, the data value may include multiple characters, numbers, bit values, or symbols. In other embodiments of the present disclosure, the data value after being labelled by the data processing module **102** may be the aforementioned record or tuple including multiple characters, numbers, bit values, or symbols.

The data values **b1** to **b5** refer to a character, a number, a bit value, or a symbol in the data unit **B** that can be recognized by the data processing module **102,** for example, Arabic numerals, Chinese characters, English characters, or symbols. For further example, if the data unit is a medical record table in an unstructured data format, the data values can be, for example: "n," "a," "m," "e," "f," "l," "o," "r," "a," " 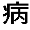 ," " 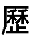 ," " 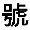 ," " 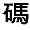 ," "1," "7," "-," "L," "c," "a," "n," "c," "e," "r." The labelled value **a1'** and the labelled value **b1'** refer to the words, values, or data of the data unit **A** and the data unit **B** after being automatically or manually labelled by the data processing module **102,** for example: "flora," "17-L," "cancer."

### Data attributes

The data attributes **DA1** to **DA3** can be "field name" and "label name" in a structured data format. More specifically, for example: "medical record number," "name," "gender," "residence," "time," "the findings/diseases," and "tumor invasion location" in order are the data attributes of the data "17-L," "Xiao-Ming(name)," "Male," "Taoyuan City," "July 15, 2021 at 10:10 AM," and "Liver Cancer," "Liver."

In some embodiments, the operating platform **101** can import the data value **a1** or the data value **b1** of the data unit **A** or the data unit **B** to the data processing module **102.** The system user can manually label the data value **a1** corresponding to a data attribute **DA1** through the operating platform **101,** and then store in the project data set as the data value **a1'.** For example, the data unit **A** in the structured data format includes data values: "job occupation", "office worker". Use symbols such as "#", "$", "%", or other characters to label before the data value a1, for example: "#job occupation". In this way, the data processing module **102** can identify "job occupation" as a data attribute.

Specifically, in some other embodiments, the operating platform **101** may further define a data attribute as a semantic labeling decision. That is, a data attribute tag is a "semantic tag" in the natural language processing. After labeling up some unstructured data formats, the unstructured data formats can be labeled with semantic tags through the data processing module.

In some embodiments, the data attribute can further provide the data processing module to be used for statistical analysis of clustering or classification. For example, the data attributes can be "residence", "marital status", "education", "age", etc., which are usually used as data attributes for clustering or classification in statistical analysis. In this way, the visualization diagram can present a diagram showing the classifications or the clusterings.

In some embodiments, as shown in FIG. 5, the data values **a1** to **a3** stored in the data unit **A** may already be corresponded respectively to the data attributes **DA1** to **DA3.** Also, in the project data set **PR,** it may have the same data attribute **DA1** and data attribute **DA2.**

In some embodiments, the data attributes may be used by the system for data processing as a basis for classification (for clustering or classification). For example, the data attribute may be "positive comment", "symptoms", "unpopular", "region", or other similar classifications or clusterings. In this way, the visualization diagram can present a diagram of classification or clustering. In other embodiments, a clustering or a classification may further include one or more clusterings or classifications. That is, the clustering or the classification can include one or more superordinate categories, and the one or more superordinate categories include one or more sub-categories. For example, the superordinate category may be "Northern of Taiwan"; the sub-categories may be "Taipei City", "New Taipei City", etc. To further explain, that is, each data attribute can include one or more data attributes.

### Project data set

The operating platform **101** can edit the project data set **PR.** Specifically, the project data set **PR** allows a system user to establish the correct data attribute corresponding to the data unit through the operating platform **101.** The project data set **PR** can create data attributes **DA1** and **DA2** in advance; or based on the data attribute **DA1** and data attribute **DA2** provided in the imported data unit **A,** it is automatically created in the project data set **PR;** alternatively, create the corresponding data attribute **DA2** to the project data set **PR** according to the data attribute which is determined by the data processing module **102** after it labeling the data unit **B.** For example:
The data attribute **DA1** is "medical record number."
The data attribute **DA2** is "discovery."
Data attribute **DA3** is "age."
The data value **a1** is "17-L."
The data value **a2** is "cervix cancer."
The data value **a3** is "36".
The data values **b1-b5** are "m", "y", "o", "m", and "a" respectively. The data processing module **102** labels the data values **b1-b5** as "the findings/diseases."
The labelled value **a1'** is "17-L". The labelled value **b1'** is "myoma", and the data processing module **102** can store the labelled value **b1'** of the "the finding/diseases" in the project data set **PR** corresponding to the data attribute **DA2.**

In some embodiments, the data processing module **102** can identify that the data unit **A** or the data unit **B** is an unstructured data format or a structured data format.

Then please refer to FIG. 6 and FIG. 5. The visualization diagram editing interface **1011** can determine a chart generating condition **1013;** the diagram generating condition **1013** includes data screening condition **1015,** diagram category **1017,** and displayed field condition **1019.** The data processing module **102** can process the labelled value **a1'** and the labelled value **b1'** according to the diagram generating condition **1013** determined by the system user, and generate the visualization diagram **103.** The operating platform **101** transmits the determined diagram generating condition **1013** to the data processing module **102** for processing, and generates a visualization diagram **103.** Each feature is described as follows:
**(1) Diagram generating condition 1013:**
   The diagram generating condition provide a graphical user interface for the system users. The diagram generating condition **1013** may include different types of conditions, which are able to determine the processing of the labelled value **a1'** and the labelled value **b1',** thereby providing a desirable diagram to the user. For example, in this embodiment, the diagram generating condition **1013** includes: a data screening condition **1015,** a diagram category **1017,** and a displayed field condition **1019.** Overall, the user can obtain the visualization diagram **103** through simple operations of the system for data process.
**(2) Data screening criteria 1015:**
   The data screening condition **1015** may further include: a data set condition **1015a,** a classification and clustering condition **1015b,** a data attribute condition **1015c,** an arithmetic unit condition **1015d,** a labelled value condition **1015e,** or a combination thereof. The data screening condition and other conditions **(1015a-1015e)** shown in FIG. 6 can be arranged in appropriate positions in a graphical user interface. In this embodiment, the conditions are presented in rows, and the rows corresponding to fields below are available for system users to fill-in or selection. The aforementioned fill-in may refer to input key word through the operating platform with a keyboard; and the selection may refer to the selection of the system user with a pull-down menu.
   The system user can edit the data screening condition **1015** through the operating platform **101** and then transmit the data screening condition **1015** to the data processing module **102.** The data processing module **102** can screen out the data contents required for generating the visualization diagram **103** according to the conditions **(1015a-1015e)** of the data screening condition **1015.** As shown in FIG. 6, the data screening condition **1015** may include a group (or row), and each group (or row) of data screening condition **1015** may include one or more combination of conditions **(1015a-1015e).** In addition, another group (row) of data screening condition **1015** can also be added. The system user can edit the relationship between each one group of data screening condition **1015** and the other through the arithmetic unit **1016,** for example: "and", "or" and other arithmetic units.
**(3) Data set condition 1015a:**
   Please refer to FIG. 5 and FIG. 6. In this embodiment, due to the project data set **PR** is named as "MRI report," the field below the data set condition **1015a** is "MRI report." In this way, system users may filter data they need. It is worth noting that, in some embodiments, there may be one or more project data sets **PR.** The system user can filter the project data set **PR** to be processed by the data processing module **102** according to the data set condition **1015a.** In addition, based on this, the data unit **A** can be one of the project data sets **PR.** In other words, because the data unit is a structured data format, and each data value **a1-a3** has the corresponding data attribute **DA1-DA3.** The operating platform **101** can establish a relationship key (Key) between the project data sets on the basis of the data attributes **DA1** - **DA3.** In this way, a relationship can be established between the data unit **A** and the project data set **PR.** The data processing module **102** can access the corresponding one or more data values according to the relationship key.
**(4) Classification and clustering condition 1015b:**
   The data attribute **DA4** can be a classification and clustering condition **1015b,** for example: positive reviews, good reviews, or other customizable classification. The operating platform **101** can label the data attribute **DA4** of the data unit **A.** In some embodiments, the data processing module **102** can automatically identify the data attribute **DA4** of the data unit. Specifically, a data unit can be classified by data co-occurrence analysis. For example: if there are positive semantic data such as "good", "healthy," etc. in a data unit at the same time, the data unit can be labelled as a "positive evaluation" clustering.
   In some embodiments, the data can be screened by the classification and clustering conditions or data attribute conditions. In this way, the system user can select the classification or the clustering to be presented in the visualization diagram by operating the operating platform.
   In other embodiments, the presentation of classifications or clustering can disclose the distribution information or summary information of different patients. Specifically, the distribution information may disclose gender ratio, area ratio, or other similar distribution information, for example, male: 40% and female: 60%. The system users can further understand the summary information of one or more "male" patients by clicking "male". It is further explained that if a point presented on the visualization diagram represents a patient, the system user, by clicking on the point, can see the summary information of the patient.
   For instance, please refer to the content revealed by the data filter condition 1015 in FIG. 6, and adjust the conditions slightly, which can be applied to the following situation: a physician would like to find ["MRI report"] [data attribute condition (in this example, the data attribute condition is "label name") equal to "findings/diseases"] ["contain"] ["cancer"] ["and"] ["MRI report"] ["data attribute condition (in this example, the data attribute condition is "label name") equal to "tumor invasion"] ["not equal to"] ["null"]. Also, the bottom left diagram category 1017, the physician selects the basic frequency table. In this way, after the visualization diagram is generated, the physician can see the "basic frequency table" and analyze it in the data set MRI report, and also know which organs cancer frequently invades (tumor invasion).
   Furthermore, to be more specific, if the data is sufficient, a part of the visualization diagram will include the location of the disclosure information of "'certain cancer' tumor invading 'certain organ tissue.‴ Wherein in the position of the disclosed information (not shown in the figures), you can click on "'a cancer" tumor invades 'a certain organ tissue‴ and see the related "clustering distribution" such as gender distribution, etc. The, the system user can click on a specific range of age, for example: "20-30 years old," and see the "patient list" or "patient summary information" to learn the medical history or information of one or more patients.
**(5) Data attribute condition 1015c:**
   The system operator can select the data attribute condition 1015c to filter the data values that needs to be processed for visualization by the data processing module **102.** As shown in FIG. 6, for example, in the field below the data attribute condition **1015c,** the selection of "findings/diseases" means to filter the one or more data values, which its data attribute as "findings/ diseases" in the project data set PR named "MRI report." In this way, the one or more data values are used as the materials for generating the visualization diagram **103.**
**(6) Diagram category 1017:**
   The diagram generating condition **1013** includes a diagram category **1017.** The diagram category includes data list, data table, basic frequency table, percentage table, co-occurrence matrix, co-occurrence list, clustering distribution, bar chart, broken line chart, table, pie chart, histogram, statistical chart, scatter chart, bubble chart, surface chart, radar chart, horizontal bar chart, timeline chart, chart of organ and body tissue, or a combination thereof. The front-end chart types also include other similar charts that can present analysis results. The so-called chart of organ and body tissue specifically refers to the data attributes displayed in the visualization diagram including at least one of "organ" or "body tissue," for example, "liver," "subcutaneous tissue," and so on.
   In some embodiments, if one or more data values corresponding to a data attribute are numerical values, the data processing module can display a visualization diagram that includes the numerical values and time in the axes. For example, the visualization diagram can display the time interval in which the data values are concentrated, or the trend of the data values in the time sequence.
**(7) Displayed field condition 1019:**
   The system user can select the data set condition **1015a** and data attribute condition **1015c** to be displayed in the visualization diagram **103.** To further illustrate, the difference between the displayed field condition **1019** and the data screening condition **1015** is that the data screening condition **1015** is for the system user to filter data through the operating platform. However, the visualized diagram **103** generated each time the system user can select different displayed field conditions **1019** according to different scenarios. For example, please refer to the displayed field condition **1019** in FIG. 6. In this embodiment, both "MRI report" and "patient data" are the project data set **PR.** After selecting "MRI report" in the field below the data set condition **1015a,** the system user can select the data attribute condition **1015c.** The same to the data screening condition **1015,** a group (row) of displayed field conditions **1019** can be added, and after selecting "patient data" in the data set condition **1015a,** the data attribute condition, "gender," can be selected.

The historical data module **107** is used to store the diagram generating condition **1013,** the visualization diagram **103,** or a combination thereof. In detail, the historical data module **107** can store the data screening condition **1015,** diagram category **1017,** and displayed field condition **1019** determined by the system user to the historical data module **107.**

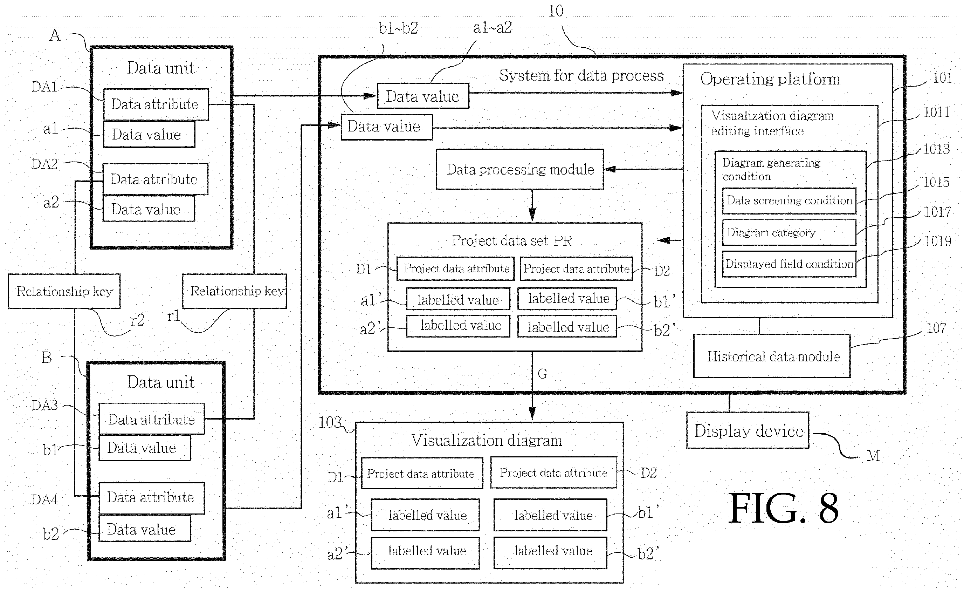

Next please refer to FIG. 8 for a block diagram of the fourth embodiment of the present disclosure. The main difference from the third embodiment is that the project data set **PR** in this embodiment includes project data attributes **D1-D2.** The operating platform **101** is further used to determine the relationship key **r1-r2;** the relationship key **r1** corresponds to the data unit **A** and the data unit **B.** To further illustrate, the relationship key **r1** corresponds to the data attribute **DA1** and the data attribute **DA3;** the relationship key **r2** corresponds to the data attribute **DA2** and the data attribute **DA4.** In this way, the data processing module **102** can generate the visualization diagram **103** according to the relationship key **r1** and the relationship key **r2.** To further illustrate, before the data processing module **102** processes the data values **a1-a2** and the data values **b1-b2** and generates the visualization diagram **103,** the data unit **A** and the data unit **B** can be established a relationship by the operating platform **101;** the so-called relationship can refer to the use of primary key in the relational database to establish the two, so that the data processing module can access the data values **a1-a2** and **b1-b2.** The project data attributes **D1-D2** of the project data set **PR** can be edited by the operating platform **101,** and then the visualization diagram 103 to be generated can be set by the visualization diagram editing interface **1011.** The content displayed in the visualization diagram **103** is mainly based on the project data attributes **D1- D2.**

In addition, the present embodiment differs from others in that both data unit **A** and data unit **B** are in a structured data format; or, data unit **B** has been labelled by the data processing module **102** and can be identified as a structured data format. In other words, the data value **b1** and the data value **b2** of the data unit **B** are corresponding to fields or data attributes.

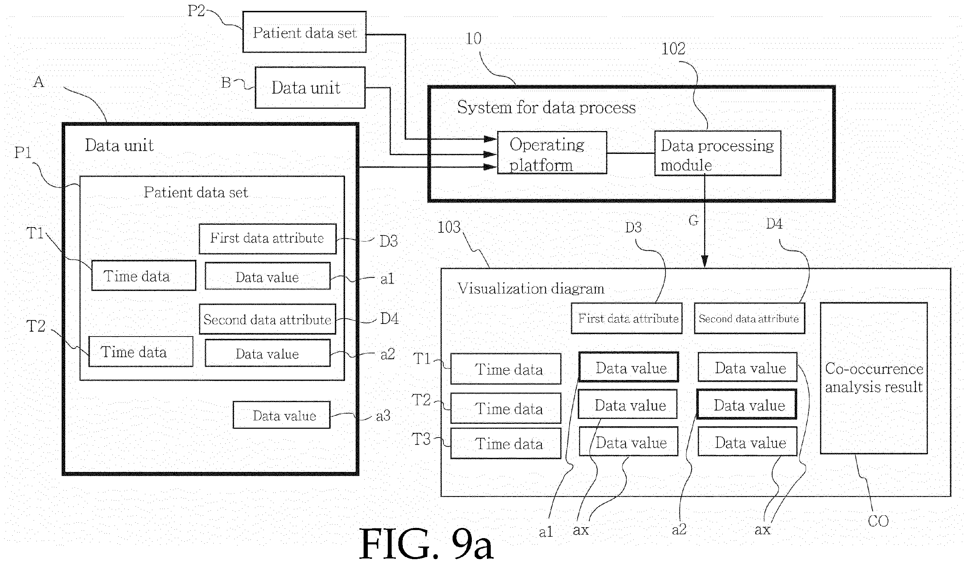

Next, please refer to FIG. 9a for a variation of the fourth embodiment of the present disclosure. The difference from the fourth embodiment is that the data unit **A** includes a patient data set **P1.** The patient data set **P1** is mainly stored in a structured data format. In some embodiments, the data unit **A** may include a data value **a3** in an unstructured data format. Similarly, the patient data set **P1** can be labelled by the data processing module **102** and be generated to a visualization diagram **103,** wherein a patient data set **P1** can store a plural of data values **a1-a2** which are respectively corresponding to a time data **T1-T2.** The data values **a1-a2** respectively correspond to the first data attribute **D3** and the second data attribute **D4.**

Wherein, the patient data set **P1** includes data values **a1-a2.** The data value **a1** corresponds to the first data attribute **D3,** and the data value **a2** corresponds to the second data attribute **D4.** The data value **a1** and the data value **a2** respectively corresponds to the time data **T1** and the time data **T2.** Time data **T1-T2** can refer to the time recorded by data values **a1-a2.** For example, the data value **a1** is "cervical cancer," the time data **T1** is "May 11, 2021," and the first data attribute is "findings/diseases."

In this variation, the patient data set **P1** can be used to analyze the condition of a patient at different points in time. For example, a patient's "findings/diseases" continue to appear "cervical cancer"", and a certain day "Uterine Fibroids" has been added to "findings/diseases." The above purpose can be accomplished by this embodiment, and please refer to FIG. 9a. A data unit A includes a patient data set **P1** as the records of a patient's condition at different points in time. The first data attribute **D3** and the second data attribute **D4** are labelled as "findings/diseases" by the data processing module **102.** (That is, in this embodiment, the first data attribute **D3** and the second data attribute **D4** can be the same or different).

The data value **a1** corresponding to the first data attribute **D3** is labelled as "cervical cancer." The time data **T1** corresponding to the first data attribute **D3** is "May 11, 2020." The data value **a2** corresponding to the second data attribute **D4** is labelled as "Uterine Fibroids," and the time data **T2** corresponding to the second data attribute **D4** is "December 11, 2020." In this way, when the data processing module **102** labels and processes the data unit **A,** a visualization diagram **103** can be generated. Please refer to the visualization diagram **103** in FIG. 9a, which contains the time data **T1** corresponding to the data value **a1;** the time data **T2** corresponding to the data value **a2.** In this embodiment, for ease of understanding, the correspondences between data attributes, time data, and data values are simply presented in a table-like manner. In other embodiments, the visualization diagram may be presented in other ways, for example, the aforementioned analytic graphs such as broken line chart, pie chart, and so on.

Another example is the following scenario: a patient's "findings/diseases" is "Uterine Fibroids," and the change in "size" of the uterine fibroids is observed during a continuous observation. Similarly, as in the mentioned embodiments, the first data attribute **D3** and the second data attribute **D4** of the patient data set **P1** are "findings/diseases." The data values **a1-a2** are numerical data. Data values **a1-a2** correspond to data time **T1-T2** respectively. When the data processing module **102** labels the data value, it can be identified as numerical data.

In order to make the data processing module **102** to identify a numerical data, the data values **a1-a2** may be manually labelled by the operating platform **101,** or may be automatically labelled by the data processing module **102.** To further explain, one may label at the front of the data value **a1** by using symbols such as "#," "$," "%," or other characters, for example: "#job occupation." In this way, the data processing module **102** can identify "job occupation" as a data attribute.

Please refer to FIG. 9a. The visualization diagram 103 not only discloses the data values **a1** and the data value **a2,** which are taken from the patient data set **P1,** but also other data valuesax, wherein the data value ax may come from another patient data set **P2** or another data unit **B** of the same patient. In order for the patient data set **P2** and the data unit **B** to correctly correspond to the data attributes, the data unit **A,** the data unit **B,** the patient data set **P1,** and the patient data set **P2** can be associated to each other by using the relationship key.

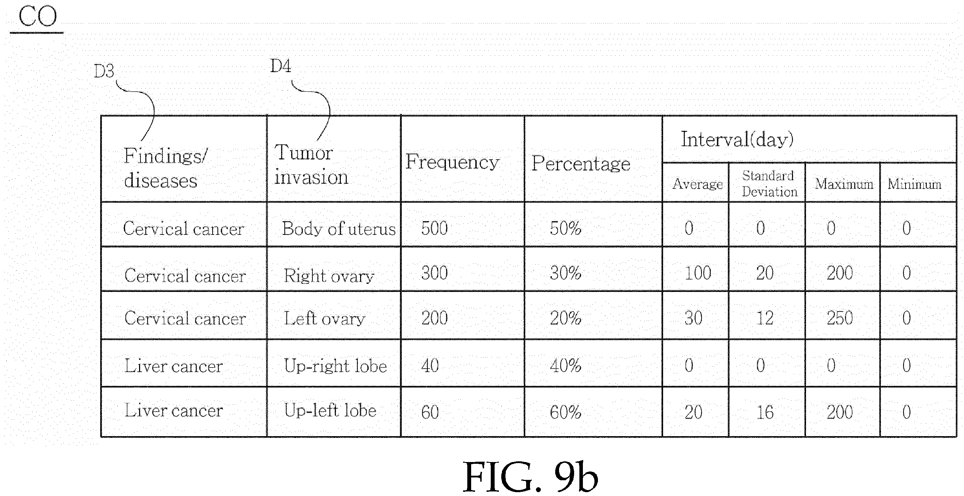

Please refer to FIG. 9a and FIG. 9b again. FIG. 9b is an example diagram showing the result of co-occurrence analysis in the fourth embodiment of the present disclosure. The visualization diagram **103** includes a co-occurrence analysis result CO. Perform co-occurrence analysis of the patient data set **P1,** the system user can know the implicit meaning of the data values. For example, the body organs often being invaded by specific cancers: cervical cancer and body of uterus often correspond to the same time data at the same time, implying that "cervical cancer" often invades "body of uterus." In addition, it is worth noting that in the previous example, the time data can also provide the system user to understand the time taken for the "cervical cancer" to invade the "body of uterus." A physician can use the co-occurrence analysis result CO to determine the body parts that need attention during surgery or examination. In addition, the physician can infer whether there is an invasion of malignant tumor, and whether to remove other body parts other than the target object in advance during the operation.

The so-called co-occurrence analysis may include analyzing data units using Pearson Product-Moment Correlation Coefficient (PPMCC); in some embodiments, Mutual Information (MI) analysis may also be applied to the data unit.

Please refer to FIG. 9b again. The co-occurrence analysis result CO may include a statistical analysis result, a probability analysis result, or a combination thereof. It is supplemented that the time data can be used to record the time of the patient's examination, the time of the patient's examination report being prepared, the time of the patient visit the hospital, the time of the patient being hospitalized, the time of the patient leave the hospital, the time of the patient's surgery start, the time of the patient's surgery end, the time of patient's birth, the time of the patient was diagnosed, the time of the patient's death, the time of a data being stored, the time of a duration, the time of the occurrence of a condition (disease/complication/symptom), the time of the end of a condition (disease/ complication/ symptom), or a combination thereof.

To add further supplemented explanation, by establishing a patient data set or establishing a relationship key; by establishing the relationship key, specifically, one or more data values of multiple data units can be corresponding to the same patient (corresponding to the same primary key). In this way, a particular patient can be analyzed. For instance, if one or more data values are presented on the visualization diagram as a kind of broken line graph, the point of the end point of the broken line graph can represent a data value in a patient data set **P1.** The system user can learn the data values, the summary or information of the data attributes stored in other data units of the patient by clicking on the point of the end point.

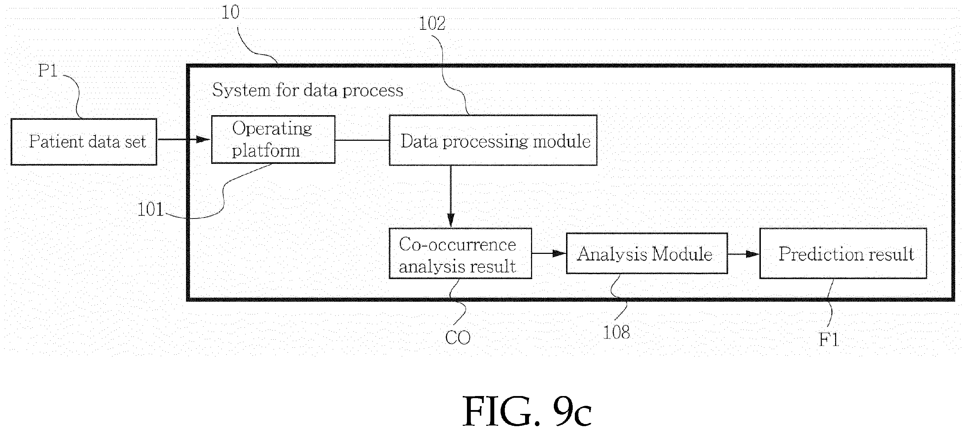

Please refer to FIG. 9c is an illustration of a variation of the fourth embodiment of the present disclosure. The system for data process **10** may include an analysis module **108** signally connected to the operating platform **101;** the analysis module **108** is used to process the co-occurrence analysis result CO, predict the patient data set, and generate a prediction result **F1.** The analysis module **108** can analyze the characteristics of data values such as density, the small-world phenomenon, degree distribution, and degree correlation. The prediction result F1 may be an evaluation to the co-occurrence analysis result **CO.** Specifically, the prediction result **F1** can be presented in a percentage, table, or other visualization approaches. In addition, the prediction result **F1** can also be described in text. For instance, please refer to the co-occurrence analysis result CO shown in FIG. 9b. When a patient is diagnosed with "cervical cancer," and the "upper right liver" and the "upper left liver" may be the location where the tumor might invade, a doctor should pay close attention to it. The special feature of this presented disclosure is that the patient data in an unstructured data format is enormous. Through this disclosed variation of the embodiment, corpus and numerical data can be quickly identified and collected, and further can be applied. In this way, we can further understand, in the results of co-occurrence analysis, whether the range of organs or body tissues and the range of time of the "tumor invasion," which are often co-occurred along with the "cervical cancer," are similar to the characteristics of other cancers.

Please refer to FIG. 10 is a block diagram of the fifth embodiment of the present disclosure. The difference between this embodiment and the embodiment disclosed in FIG. 2 is that the data processing module **102** can determine a correlation judgement **R1.** The data processing module **102** can further generate the visualization diagram **103** according to the correlation judgment **R1.** The data unit **A** and the data unit **B** includes a plural of data values **a1-a3** and a plural of data values **b1-b3.** The data processing module **102** judges the data value **a1** included in the data unit **A** and the data value **b1** included in the data unit **B** a correlation judgment **R1;** or, the data value **a1** and the data value **b2,** or the data value **a1** and the data value **b3.** Similarly, the correlation judgment can also be any two of the data values **a1-a3** included in the data unit A. Correlation judgment **R1** includes: "greater than," "equal to," "less than," "not equal to," "greater than or equal to," "including," "less than or equal to," or other similar logical judgement operators. The correlation judgment **R1** can be set by the operating platform to determine the judgment conditions, for example: "greater than the value 0," "less than or equal to the value 2." The data processing module **102** generates a visualization diagram **103** further according to the correlation judgment **R1.** For example, if the data value is time data, the time sequence can be judged. If the data value is numerical data, the size can be judged and sorted. The content disclosed above can also be displayed on the visualization diagram **103.**

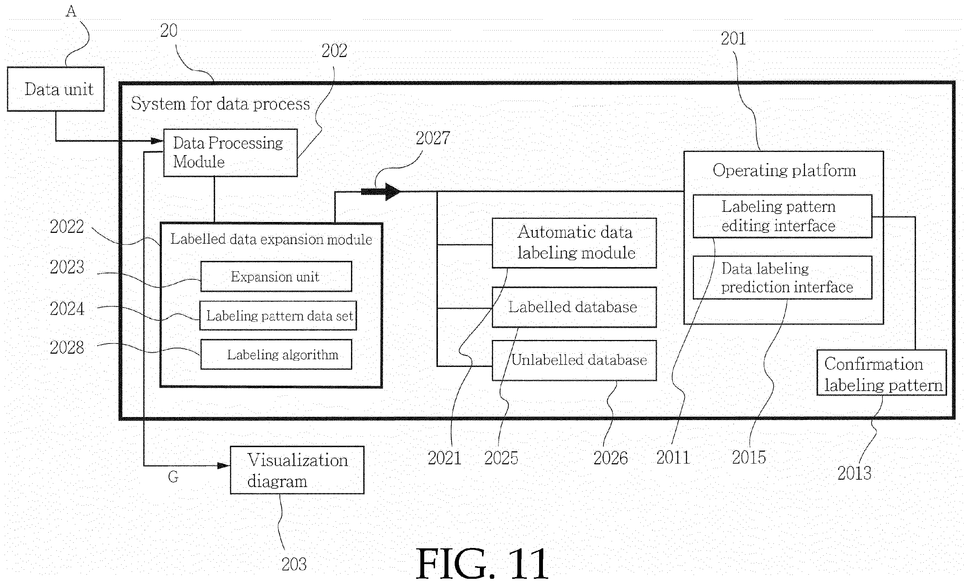

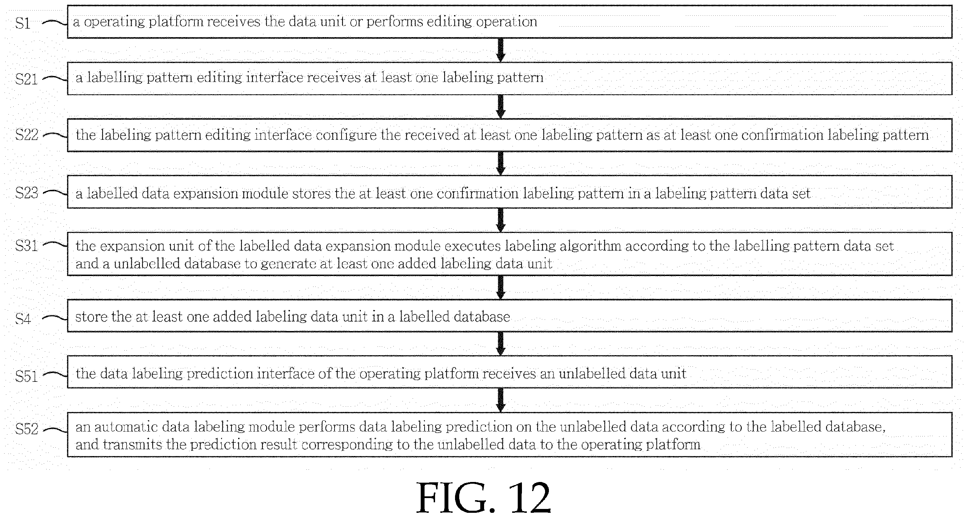

Next please refer to Fig. 10 and the sixth embodiment disclosed in Figs. 11 and 12 described later. This present disclosure can also predict the labeling result of the data processing module. That is, when the data value is a word, numerical data, or other characters, the present disclosure can determine the data value with similar semantic meaning and correspond to the correct data attribute. Specifically, for example, the wording difference between "cervical cancer" and "cervikal cancer" can be judged by the prediction result and the correct data attribute can be labelled as "findings/diseases."

The correlation judgment may further include logical operation. Specifically, for instance, the labelled data values of data unit **A** and data unit **B** are all numerical values, which can determine whether the data values to be used to generate the visualization diagram are redundant or conflicting. For example, a1=30, b1=30, the correlation judgment **R1** can be "intersection." In this way, the data unit **A** and the data unit **B** can be combined, and used for the data processing module 102 to generate the visualization diagram **103.**

In some embodiments, the data unit being processed as the project data set is visualized by the data processing module. The project data set defines the data attributes by the operating platform, which the data attributes are processed and generated by the data processing module. In this way, by using the correlation judgment **R1** to judge the relationship of the data values between each other, the data processing module can be used to copy, move, or delete the data values of the data unit and the data values of the project data set.

Next please refer to FIG. 11, which is a block diagram I of the sixth embodiment of the present disclosure. This embodiment mainly provides an embodiment of a data processing module **202** labeling the data unit **A.** The data processing module **202** further includes a labelled data expansion module **2022.** The system for data process **20** further includes a labelled database **2025,** an unlabelled database **2026,** an automatic data labeling module **2021** signally connected to the labelled database **2025,** and an operating platform **201** signally connected to the labelled database **2025,** the unlabelled database **2026,** and the labelled data expansion module. The labelled data expansion module **2022** can access the unlabelled database **2026** and the labelled database **2025,** and includes a labeling pattern data set **2024** being able to store a labeling pattern and an expansion unit **2023** being able to execute a labeling algorithm **2028.**

The labelled database **2025** is used to store labelled data units, and the unlabelled database **2026** is used to store unlabelled data units.

The operating platform **201** includes a labeling pattern editing interface **2011** and a data labeling prediction interface **2015,** and the labeling pattern editing interface **2011** can be used to input data and perform editing operations to generate at least one confirmation labeling pattern **2013.** The operating platform **201** of this embodiment is provided for system users to input data and perform editing, adding, and deleting. In addition, the operating platform **201** can input data and perform editing, adding, and deleting by an application program interface (API). The data labeling prediction interface **2015** can be used to input data and display forecast results. The automatic data labeling module **2021** of this embodiment can be configured to perform data labeling prediction.

The labelled data expansion module **2022** performs operations to generate at least one added labeling data unit **2027** according to at least one confirmation labeling pattern **2013** and unlabelled database **2026,** and stores the at least one added labeling data unit **2027** in the labelled data database **2025** in order to expand the labelled database **2025.** The data processing module **202** is used to process the added labeling data unit **2027** and generate a visualization diagram **203.**

Please further refer to FIG. 12, which is a flowchart of a method for data labeling according to the sixth embodiment of the present disclosure, and is applicable to the system for data process **20** shown in FIG. 10. The steps of the method for data labeling of the sixth embodiment are described as follows: performing step **S1,** the labeling pattern editing interface **2011** of the operating platform **201** receives the data unit **A** or perform editing operation, and perform step **S21,** the labelling pattern editing interface **2011** receives at least one labeling pattern. Then, performing step **S22,** the labeling pattern editing interface **2011** configure the received at least one labeling pattern as at least one confirmation labeling pattern **2013.** In this embodiment, the system user performs editing on the labeling pattern editing interface **2011** to input the labeling pattern, and the labeling pattern editing interface **2011** uses the labeling pattern inputted by the system user as the confirmation labeling pattern **2013.** It is supplemented that, in other embodiments, the labeling pattern editing interface **2011** may also receive the input of data unit **A** or perform editing, adding, and deleting via an external application program interface.

After obtaining the at least one confirmation labeling pattern **2013,** perform step **S23,** and the labelled data expansion module **2022** stores the at least one confirmation labeling pattern **2013** in a labeling pattern data set **2024.** In this embodiment, labelled data expansion module **2022** first performs a test according to the at least one confirmation labeling pattern **2013** and the labeling pattern in the labeling pattern data set **2024;** then, after the test is confirmed, the at least one confirmation labeling pattern **2013** is stored and updated to the labeling pattern data set **2024.** The labelled data expansion module **2022** tests whether there is a repetition or a conflict between the at least one confirmation labeling pattern **2013** and the data unit in the labeling pattern data set **2024.** In this way, the repetition or the conflict between the labeling patterns can be removed. In other embodiments, the expansion unit **2023** may also perform the aforementioned test.

Then perform step **S31,** the expansion unit **2023** of the labelled data expansion module **2022** executes labeling algorithm **2028** according to the labelling pattern data set **2024** and an unlabelled database **2026** to generate at least one added labeling data unit **2027;** and perform step **S4** to store the at least one added labeling data unit **2027** in a labelled database **2025.** Specifically, in step **S31** of this embodiment, the expansion unit **2023** is based on the at least one confirmation labeling pattern **2013** in the labeling pattern data set **2024** and the labeling patterns that has been stored in the labeling pattern data set **2024,** to execute the labeling algorithm **2028** on the data unit in the unlabelled database **2026** in order to label the data unit in the unlabelled database **2026,** and the at least one added labeling data unit **2027** is generated. After performing step **S31,** the expansion unit **2023** stores the generated added labeling data unit **2027** in the labelled database **2025** to expand the labelled database **2025.** The labeling algorithm **2028** of this embodiment may be a string searching algorithm or a maximum matching algorithm.

In the case that there is no pre-existing data unit in the labeling pattern data set **2024,** step **S23** of this embodiment may optionally not be performed. In this case, in step **S31,** the expansion unit **2023** labels the data unit in the unlabelled data base **2026** according to the at least one confirmation labeling pattern **2013** generated in the step **S22.**

The method for labeling data unit of the data processing module **202** in this embodiment can further perform step **S51,** that the data labeling prediction interface **2015** of the operating platform **201** receives an unlabelled data unit; and step **S52** is performed, that an automatic data labeling module **2021** performs data labeling prediction on the unlabelled data according to the labelled database **2025,** and transmits the prediction result corresponding to the unlabelled data to the operating platform **201.** The automatic data labeling module **2021** of this embodiment may execute algorithms such as Recurrent Neural Network, Conditional Random Field, and Maximum-Entropy Markov Model. Then, the operating platform **201** displays the prediction result corresponding to the unlabelled data.

The system for data process **20** shown in FIG. 11 and FIG. 12 can be used to process corpus data, image data, or audio data. In the case of processing corpus data, the unlabelled database **2026** is a corpus database, and the labelled database **2025** is a labelled corpus database, that is, the labelled corpus data is stored. In addition, the unlabelled data is corpus data. The confirmation labeling pattern is used to label corpus data, which may include at least one of morphological information, syntax information, and semantic information.

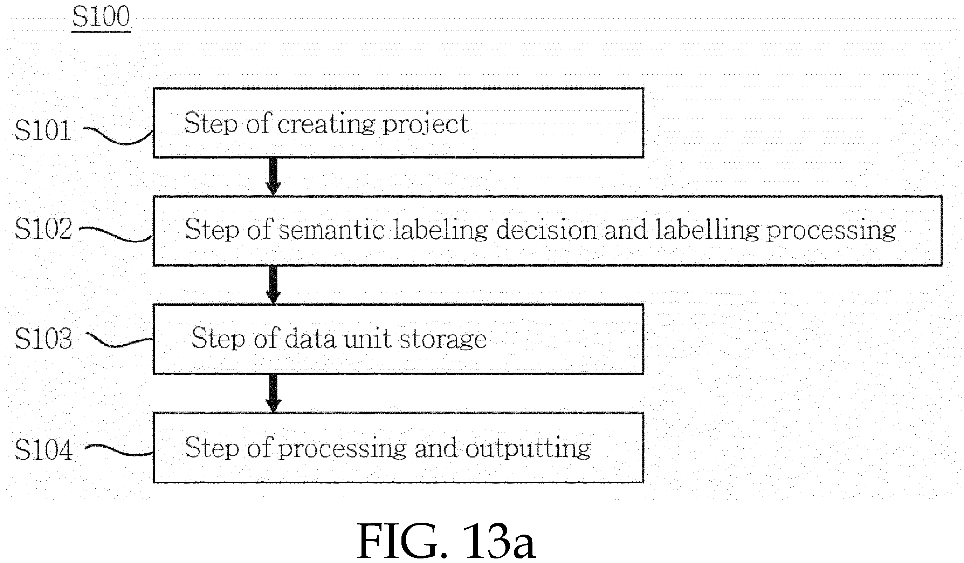

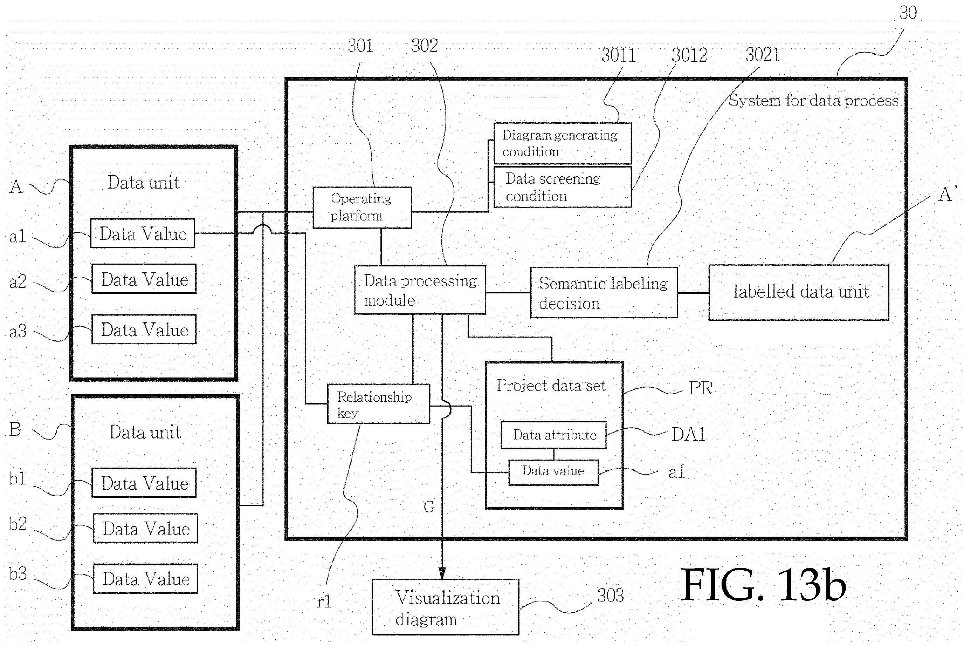

Next, please refer to FIG. 13a and FIG. 13b for the block diagram and flowchart of a system for data process **30** and a method for data process **S100** of the present disclosure. The steps are described as follows: perform step **S101** of creating project. A project data set **PR** is generated by the operating platform 101 to access at least one data unit **A** including a plural of data values **a1-a3.** The project data set **PR** includes at least one data attribute **DA1.**

Perform step **S102** of semantic labeling decision and labeling processing. The data processing module **102** processes at least one data unit **A** and determines a semantic labeling decision **3021,** and outputs a labelled data unit **A'.** The semantic labeling decision **3021** makes one of the plural data values **a1-a3** correspond to at least one data attribute **DA1.** When the at least one data unit **A** is in an unstructured data format, the data processing module **302** performs an automatic semantic labeling step to complete the semantic labeling decision **3021** so that the data value **a1** corresponds to the data attribute **DA1.** Perform step **S103** of data unit storage. The data processing module **302** stores the labelled data unit **A'** to the project data set **PR.** Perform step **S104** of processing and outputting. The data processing module **302** processes the project data set **PR** to generate a visualization diagram **303.**

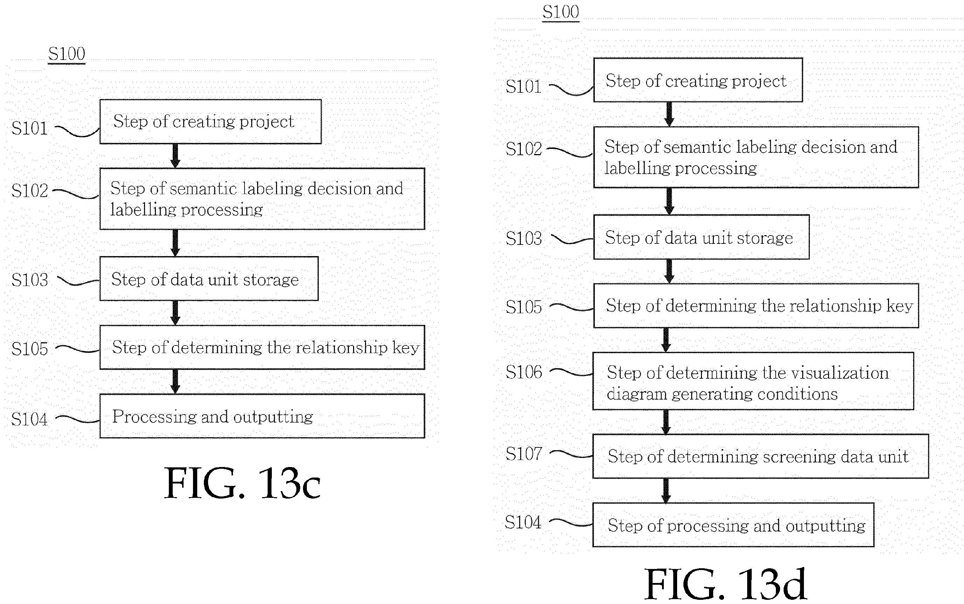

Please refer to FIG. 13c for a variation of the method for data process **S100.** The method for data process **S100,** wherein the system for data process **30** further includes a plurality of data units **A** and data units **B.** Before step **S104** is performed, it includes step **S105** of determining the relationship key: the data processing module **302** determines a relationship key **r1.** The relationship key **r1** determines that the data value **a1** corresponds to the data attribute **DA1** of the project data set **PR.**

Please refer to FIG. 13d for another variation of the method for data process **S100.** The method for data process **S100** further includes the step **S106** of determining the visualization diagram generating conditions: the operation platform is connected to the data processing module, the operation platform **301** determines a diagram generating condition, and the data processing module **302** performs step **S104** after screening the project data set according to the diagram generating condition.

Please refer to FIG. 13d again, the method for data process may further include step **S107** of determining screening data unit: the operating platform **301** determines a data screening condition **3012,** and the data processing module **302** screens the data unit **A** according to the data screening condition **3012,** and then performs step **S103** and step **S104.**

The above are only preferred embodiments of the present invention, and are not used to limit the scope of embodiment of the present invention; anyone who is familiar with this technique and makes equal changes and modifications without departing from the spirit and scope of the present invention shall be covered by the claims of this disclosure.

To sum up, the present disclosure obtains the patent requirements "utility," "novelty" and "non-obviousness"; the applicant filed an application for a patent before the Patent Office in accordance with the provisions of the Patent Law.

## Claims

1. A system for data process, comprising:
an operating platform for storing and reading a data unit;
a data processing module, signally connected to the operating platform; and the data unit is structured or unstructured;
wherein the data processing module labels and processes the data unit, and generates a visualization diagram.

2. The system of claim 1, further comprises:
an unstructured database;
a structured database;
the operating platform signally connected to the unstructured database and the structured database;
wherein at least one of the unstructured database and the structured database storing at least one of the data unit; and
the data processing module labeling and processing at least one of the data unit, and generating the visualization diagram.

3. The system of claim 2, wherein the unstructured database stores at least one of the data unit, and the structured database stores at least one another data unit.

4. The system of claim 2 or 3, wherein the data processing module further used for automatically labeling the data unit; and
when the data unit is unstructured, the data processing module automatically labeling and processing the data unit;
wherein the data processing module determines whether to automatically label the at least one data unit according to which of the database the at least one data unit belongs:
when the at least one data unit is stored in the unstructured database, the data processing module automatically labels and processes the at least one data unit, and generates the visualization diagram; and
when the at least one data unit is stored in the structured database, the data processing module processes and generates the visualization diagram.

5. The system of one of claims 1 to 4, wherein the data processing module is further used to process the at least one data unit and generates a visualizable data set;
wherein the data processing module generates the visualization diagram according to the visualizable data set.

6. The system of claim 5, wherein the operating platform further used to import the visualizable data set, which is generated by the data processing module pre-processed the at least one data unit; the data processing module generates the visualization diagram according to the visualizable data set being imported.

7. The system of claim 5 or 6, further comprising a visualizing module signally connect to the operating platform and the data processing module; wherein the visualizing module generates the visualization diagram according to the visualizable data set.

8. The system of one of claims 1 to 7, the operating platform comprises a visualization diagram editing interface used to configure the generating conditions of the visualization diagram and decide a diagram generating condition;
wherein the data processing module processes the at least one data unit and generates the visualization diagram according to the diagram generating condition.

9. The system of one of claims 1 to 8, wherein the at least one data unit comprises at least one data value;
wherein the operating platform is used to generate a project data set, including at least one project data attribute;
wherein the operating platform determines that the at least one project data attribute corresponds to the data attribute, which the at least one data value belongs to; and
the data processing module generates the visualization diagram according to the project data set.

10. The system of claim 9, wherein the project data set comprises:
a plurality of project data attributes, and the at least one data unit including a plurality of data values;
wherein the operating platform determines:
one of the plurality of project data attributes corresponds to the data attribute one or more than one of the plurality data values belong to; or more than one of the plurality of data attributes correspond to the data attribute one or more than one of the plurality of data values belong to.

11. The system of one of claims 1 to 10, further comprises a plurality of data units, and any one of the plurality of the data units includes at least one data value;
wherein the operating platform is further used to determine at least one relationship key;
wherein the relationship key corresponds to one of the plurality of data units and another one of the plurality of data units; and
wherein the data processing module further generates the visualization diagram according to the at least one relationship key.

12. The system of one of claims 1 to 11, wherein the data unit comprises: a patient data set, including a plurality of data values; the data process module processes the patient data set to generate the visualization diagram;
wherein any one of the plurality of data values corresponds to at least one data attribute, the data processing module generates the visualization diagram according to the plurality of data values and the data attribute; wherein the visualization comprises representations of the at least one data attribute.

13. The system of claim 12, wherein the data attribute comprises a plurality of time data, a first data attribute, and a second data attribute;
one of the plurality of data values corresponds to the first data attribute and one of the plurality of time data;
another one of the plurality of data values corresponds to the second attribute and the one of the plurality of time data;
wherein the data processing module generates a co-occurrence analysis result according to the one of the plurality of time data, the one of the plurality of data values, and the another one of the plurality of data values; the visualization diagram comprises the co-occurrence analysis result.

14. The system of claim 13, comprises an analysis module signally connected to the operating platform; the analysis module used to process the co-occurrence analysis result, predict the patient data set, and generate a predicted result.

15. The system of claim 13 or 14, the co-occurrence result further comprising
statistical analysis result, probability analysis result, or a combination thereof.

16. The system of claim 15, the plurality of time data comprises a time record of patient's examination, a time record of patient's examination report being made, a time record of patient's visit a doctor, or a combination thereof.

17. The system of one of claims 1 to 16, further comprising: a labeling database and an unlabelling database;
wherein the data processing module comprises a labelled data expansion module;
the operating platform signally connected to the labeling database, the unlabeling database and the labelled data expansion module, and comprised a labeling pattern editing interface, and the labeling pattern editing interface used for inputting data units and performing editing operation to generates at least one confirmation labeling pattern;
wherein the labelled data expansion module performs operation to generates at least one added labeling data unit according to the at least one confirmation labeling pattern and the unlabeling database, and restores the at least one added labeling data unit to the labeling database;
wherein the data processing module used for processing the added labelled data unit, and generating a visualization diagram.

18. A method for data process, a data processing module is used to process one of unstructured data and structured data, to process at least one data unit, and perform the following steps:
(A) a step for project creation: an operating platform generates a project data set for accessing the at least one data unit including a plurality of data values; the project data set includes at least one data attribute;
(B) a step for semantic labeling decision and labeling process: the data processing module processes the at least one data unit, determines a semantic labeling decision, and outputs a labelled data unit, the semantic labeling decision makes one of the plurality of data values to correspond to the at least one data attribute;
when the at least one data unit is unstructured data format, the data processing module performs a step for automatic semantic labeling to complete the semantic labeling decision makes one of the plurality of data values to correspond to the at least one data attribute;
(C) a step for data unit storage: the data processing module stores the labelled data unit to the project data set;
(D) a step for determining a relationship key: the data processing module determines a relationship key, and the relationship key determines one of the plurality of data values of one of the plurality of data units corresponding to one of the at least one data attribute of the project data set; and
after the step of (D) is executed, comprising (E) a step for process and output: the data processing module processes the project data set to generate a visualization diagram.

19. The method of claim 18, further comprises:
(F) determining visualization diagram generating condition, an operating platform is connected to the data processing module, the operating platform determines a diagram generation condition, and after the data processing module screening the project data set according to the diagram generation condition, executes the step of (D).
